# FASCICULE DE BREVET EUROPEEN

(11) **EP 1 330 987 B1**
(45) Date de publication et mention de la délivrance du brevet: **09.03.2005**
(21) Numéro de dépôt: 02356259.8
(22) Date de dépôt: 12.12.2002
(51) Int. Cl.: A61B 17/70

(54) **Implant vertebral inter-épineux**
Zwischen Wirbeldornfortsätzen eingesetzte Wirbelsäulenimplantat
Interspinous vertebral implant

(30) Priorité: 28.01.2002 FR 0200977
(43) Date de publication de la demande: 30.07.2003
(73) Titulaire: INDUSTRIAS QUIRURGICAS DE LEVANTE S.L., 46988 Fuente del Jarro (ES); Elberg, Jean-François, 92200 Neuilly sur Seine (FR)
(72) Inventeur: Elberg, Jean-François, 92200 Neuilly sur Seine (FR); de Witte, Gérard, 26000 Chateauneuf sur Isere (FR)
(74) Mandataire: Vuillermoz, Bruno

(56) Documents cités:
- WO-A-99/40866
- FR-A- 2 681 525
- FR-A- 2 717 675
- FR-A- 2 722 980

## Description

L'invention concerne un implant vertébral inter-épineux, destiné à faire fonction de stabilisateur entre deux vertèbres consécutives. Plus précisément, un tel implant est destiné à être positionné entre les apophyses épineuses de deux vertèbres consécutives.

L'invention trouve directement son application dans le cadre des phénomènes dits de déstabilisation vertébrale. Ces phénomènes se traduisent par des mouvements anormaux de la colonne vertébrale, plus particulièrement au niveau lombaire, se matérialisant par des douleurs appelées lombalgies.

Si certaines de ces lombalgies peuvent être traitées par kinésithérapie, d'autres en revanche revêtent une forme plus définitive, invalidant de manière drastique et rédhibitoire le patient qui en est affecté. Ces lombalgies peuvent également résulter de la dégradation ou de la dégénérescence du disque inter-vétébral, qui peut aboutir à un jeu anormal des vertèbres attenantes à ce disque.

Afin de remédier à ces problèmes d'instabilité, il a tout d'abord été proposé de procéder à une arthrodèse, c'est à dire à un blocage mécanique des vertèbres consécutives concernées. Pour ce faire, sont mis en place des éléments rigides constitués le plus souvent de tiges métalliques, implantées le long de plusieurs vertèbres de part et d'autre des apophyses épineuses. Les moyens d'ancrage osseux de ces tiges sont généralement constitués par des vis dites pédiculaires implantées au niveau des pédicules vertébrales.

Ce type de dispositif présente tout d'abord l'inconvénient d'être relativement difficile à implanter, nécessitant un travail de mise en place important et complexe.

En outre, il immobilise un segment vertébral relativement long, ce qui réduit notablement la mobilité du patient et peut soumettre les articulations vertébrales situées de part et d'autre de ce segment rigide, à des contraintes importantes susceptibles de générer de nouvelles instabilités à ce niveau.

On a également proposé pour remédier à ces phénomènes d'instabilité la mise en place de ligaments, également implantés au niveau de vis pédiculaires.

On s'est malheureusement heurté au problème de la relative fragilité de ces ligaments, outre au fait qu'ils ne travaillent que dans le sens de l'extension, et non pas de la compression. De fait, ils ne permettent pas de faire face de manière efficace et durable aux instabilités précitées.

On a alors proposé de mettre en place une cale insérée entre les apophyses épineuses des vertèbres concernées. La fixation d'une telle cale à ce niveau met en oeuvre des ligaments tissulaires, par exemple réalisé en DACRON (marque déposée) venant entourer les apophyses voisines.

Outre la complexité de mise en place d'une telle cale, la nécessité de faire passer le ligament autour des apophyses implique une intervention au niveau de zones saines pour aménager un passage et ensuite affaiblir les ligaments naturels.

Il a également été proposé, par exemple dans le document WO99/40866 qui constitue l'état de la technique le plus poche, un stabilisateur inter-épineux, comportant un corps compressible dans la direction du rachis, destiné à être inséré entre deux apophyses épineuses consécutives, et muni d'organes d'ancrage au niveau des apophyses épineuses des deux vertèbres respectives, voir le préambule de la revendication 1.

Si certes, cet implant permet de pallier les différents inconvénients précités tout en assurant la fonction de stabilisation recherchée, en revanche, l'expérience démontre qu'en cas de flexion de la colonne, notamment au niveau lombaire, ledit implant est susceptible d'être éjecté hors de son lieu de mise en place : en d'autres termes, sa fixation au niveau de la zone inter-épineuse n'est pas suffisante pour éviter ce genre d'écueil.

L'objet de l'invention vise un implant inter-épineux, susceptible de permettre une stabilisation à tout le moins locale du rachis, tout en évitant l'ensemble des inconvénients précédents.

Cet implant vertébral inter-épineux comporte un corps destiné à venir s'insérer entre deux apophyses inter-épineuses consécutives, compressible selon la direction du rachis, et muni de moyens d'ancrage au niveau d'au moins une apophyse épineuse.

Il se caractérise en ce que le corps est constitué d'une boucle simple refermée sur elle-même, et en ce que lesdits moyens d'ancrage sont constitués de deux pattes de fixation, solidarisées au corps, s'étendant de part et d'autre de l'apophyse épineuse au niveau de laquelle ils sont destinés à venir s'ancrer lorsque l'implant est en place, et sont percés d'orifices traversants, selon une direction sensiblement perpendiculaire au plan général desdites apophyses, les orifices traversants étant destinés à recevoir des plots de fixation pour être sertis au niveau de l'apophyse considérée.

Ce faisant, l'implant stabilisateur inter-vertébral conforme à l'invention, autorise une certaine mobilité de deux vertèbres au niveau duquel il est implanté l'une par rapport à l'autre, en reproduisant partiellement la biomécanique d'un disque inter-vertébral sain.

Un tel implant permet en outre la flexion ou l'extension du rachis, et ne nécessite pas une intervention chirurgicale lourde pour sa mise en oeuvre.

Selon une caractéristique avantageuse de l'invention, le corps intervétébral est constitué d'une lame ressort fermée sur elle-même, sensiblement en forme de 8 ou de haricot, et symétrique par rapport au plan médian.

Les organes d'ancrage sont constitués par des pattes de fixation, également symétriques l'une de l'autre par rapport au plan médian précité, voire même parallèle l'une à l'autre, lesdites pattes de fixation présentant au voisinage de leur extrémité libre, un orifice traversant, de forme tronconique, à l'instar d'un cône morse, propre à coopérer avec des plots d'ancrage de forme complémentaire, assurant ainsi leur autorétention à ce niveau, une fois les plots sertis dans l'apophyse épineuse.

Selon une première forme de réalisation de l'invention, le corps inter-épineux comprend deux jeux de deux pattes de fixation pour permettre ainsi la fixation de l'implant vertébral inter-épineux au niveau des apophyses correspondantes de deux vertèbres consécutives.

Selon une autre variante de l'invention, les organes de fixation comprennent d'une part, deux pattes de fixation au niveau d'une apophyse inter-épineuse, et d'autre part, un arc divergent à partir dudit corps, dont chacune des extrémités libres comporte un orifice traversant permettant le passage à ce niveau de vis pédiculaires ou vis de fixation d'ostéosynthèse.

La manière dont l'invention peut être réalisée et les avantages qui en découlent ressortiront mieux des exemples de réalisation qui suivent, donnés à titre indicatif et non limitatif, à l'appui des figures annexées.
La figure 1 est une représentation schématique en perspective partielle du dispositif conforme à l'invention mis en place au niveau de deux vertèbres lombaires consécutives.
La figure 2 est une représentation schématique en section partielle de la figure 1.
La figure 3 est une représentation schématique en perspective de l'implant vertébral inter-épineux conforme à une première forme de réalisation de l'invention.
La figure 4 est une représentation schématique en section transversale de l'implant vertébral inter-épineux de la figure 3.
La figure 5 est une vue analogue à la figure 4, dans laquelle l'implant présente des dimensions différentes.
La figure 6 est une représentation schématique en perspective d'un plot de fixation de l'implant conforme à l'invention.
La figure 7 est une représentation schématique vue de face de l'implant conforme à l'invention, muni de plots de fixation.
La figure 8 est une représentation schématique vue de face de l'implant conforme à une autre forme de réalisation de l'invention.
La figure 9 est une représentation schématique, visant à illustrer la mise en oeuvre de l'implant de la figure 8.

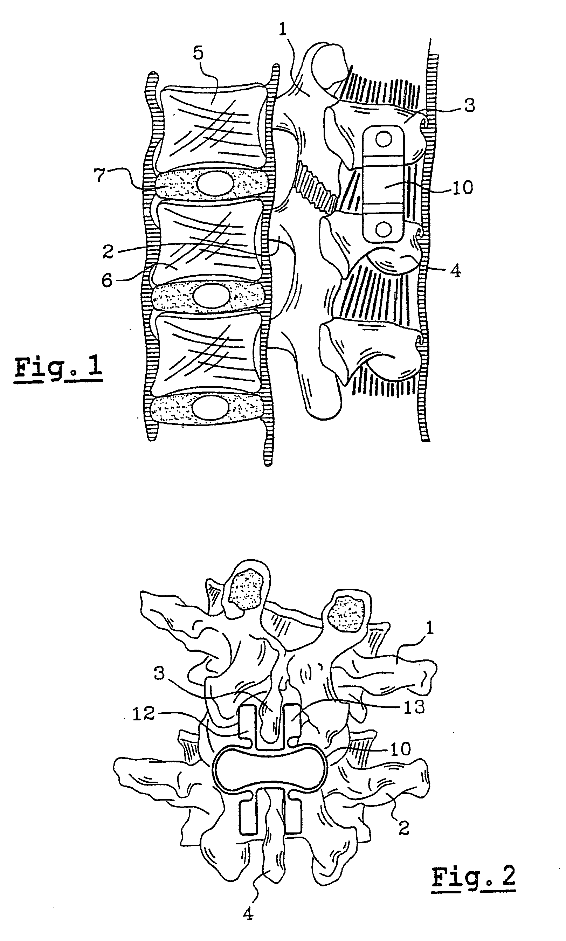

On a représenté en relation avec les figures 1 et 2 l'implant vertébral inter-épineux conforme à l'invention, mis en place au niveau de deux vertèbres lombaires consécutives. Ces vertèbres lombaires portent les références générales (1) et (2), au niveau desquelles a été représentée notamment l'apophyse épineuse respectivement (3) et (4), le corps vertébral respectivement (5) et (6), ainsi qu'un disque intervertébral (7).

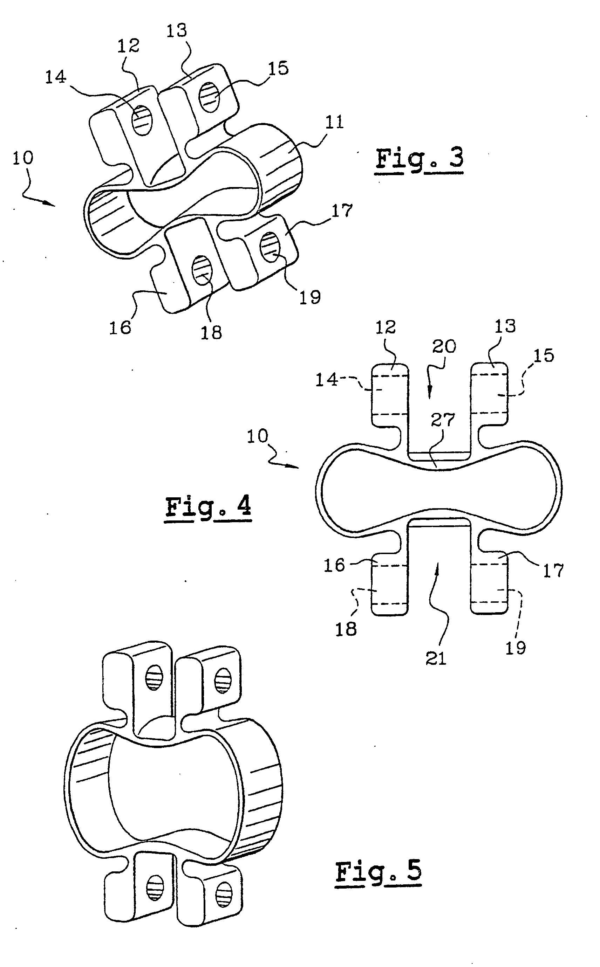

Ainsi qu'on peut bien l'observer sur ces figures 1 et 2, l'implant (10) de l'invention est destiné à venir s'insérer entre deux apophyses épineuses consécutives (3) et (4). Cet implant est plus particulièrement décrit en relation avec les figures 3 et 4.

Il est fondamentalement constitué d'un corps (11), constitué d'une lame ressort fermée sur elle-même, en forme de 8 non fermé en son centre ou de haricot. Cette lame ressort confère donc à l'implant un certain degré de compressibilité, degré qui peut être modulable en fonction de l'épaisseur donnée à la lame ressort qui le constitue.

De part la configuration particulière du corps et de par sa mise en place au niveau du rachis lombaire, la direction de la compressibilité est sensiblement alignée avec celle du rachis, et ce de telle sorte à permettre tant les moments de compression que d'extension du rachis lorsque l'implant est positionné entre les apophyses épineuses.

Ce corps (11) est symétrique par rapport au plan médian vertical. Il est avantageusement réalisé en titane ou en un alliage de titane de type TA6V, c'est à dire un alliage de titane comportant 6% en poids d'aluminium, et 4% en poids de vanadium.

Le corps présente de part et d'autre de ses deux faces principales des pattes d'ancrage, respectivement (12), (13) et (16) et (17), les deux pattes de chacun des jeux étant symétriques par rapport au plan médian précité. La forme du corps peut être adaptée en fonction du lieu d'implantation du stabilisateur inter-vertébral, en jouant sur sa configuration particulière, et notamment toujours en forme de 8 ou de haricot, ainsi que sur ses dimensions, tel que par exemple on peut l'observer sur la figure 5.

En l'espèce, ces pattes d'ancrage s'étendent selon deux directions parallèles l'une à l'autre, et définissent un espace libre respectivement (20, 21), permettant le passage à ce niveau des apophyses épineuses des vertèbres considérées, ainsi qu'on peut l'observer en figure 1. Les deux pattes (12, 13) d'un même organe d'ancrage s'étendent donc en regard l'une de l'autre. Chacune de ces deux pattes présente au voisinage de son extrémité libre un orifice traversant, respectivement (14) et (15), en forme de cône morse.

Ces orifices traversants sont orientés sensiblement perpendiculairement au plan général contenant l'apophyse épineuse, tel qu'on peut par exemple s'en rendre compte sur la figure 2. Il en est de même s'agissant des pattes (16) et (17), munies d'orifices traversants, également en forme de cône morse, respectivement (18) et (19).

L'ensemble de l'implant est monobloc et forgé d'une seule pièce, à base d'alliage de titane tel que précédemment mentionné.

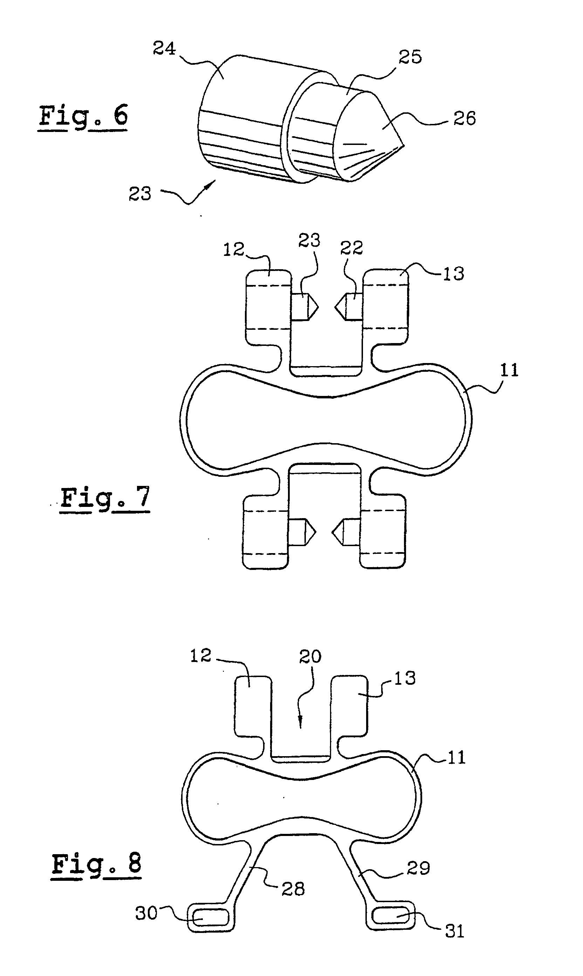

Les orifices (14), (15), (18) et (19) sont destinés à coopérer chacun avec un plot de fixation (23), tel que représenté en figure 6.

Ce plot de fixation comporte donc tout d'abord une zone d'insertion (24), également en forme de cône morse et de forme complémentaire à la forme interne des orifices traversants (14, 15, 18 et 19). Cette portion en forme de cône morse (24) se prolonge en une zone cylindrique (25), de diamètre inférieur, se terminant par un profilé en forme de pointe (26), afin de permettre le sertissage dudit plot dans l'apophyse épineuse, et corollairement la fixation de l'implant.

De fait, lorsque les plots sont insérés au niveau des orifices précités, seules les zones (25) et (26) émergent dans l'espace (20) (21) entre les pattes, tel qu'on peut l'observer en figure 7.

Cet implant présente donc un profil anatomique et, s'avère d'un certain confort pour le patient. Afin d'optimiser ce profil anatomique, l'implant présente une certaine surépaisseur (27) au voisinage des zones de raccordement des pattes (12, 13, 16, 17) sur le corps (11)

L'implant conforme à l'invention peut être inséré par simple engagement dans l'espace inter-épineux entre les deux vertèbres adjacentes considérées, ne nécessitant qu'une intervention limitée tant au niveau invasif qu'au niveau temps, puisqu'il suffit d'écarter légèrement les apophyses épineuses pour permettre sa mise en place au moyen d'un ancillaire adapté.

En outre, un tel implant ne subit aucune usure, puisqu'il n'est pas soumis à des frottements répétés. De la sorte, il peut être maintenu en place pendant de nombreuses années, sans nécessiter son remplacement.

De plus, de par son principe de fixation, on conserve la mobilité relative des deux vertèbres concernées, tant en compression qu'en extension, mais également en mouvement relatif des vertèbres concernées l'une par rapport à l'autre selon une direction perpendiculaire à l'axe du rachis, dans la mesure où le mode e fixation par plots fait fonction en quelque sorte d'axe d'articulation, autorisant de fait un tel déplacement relatif.

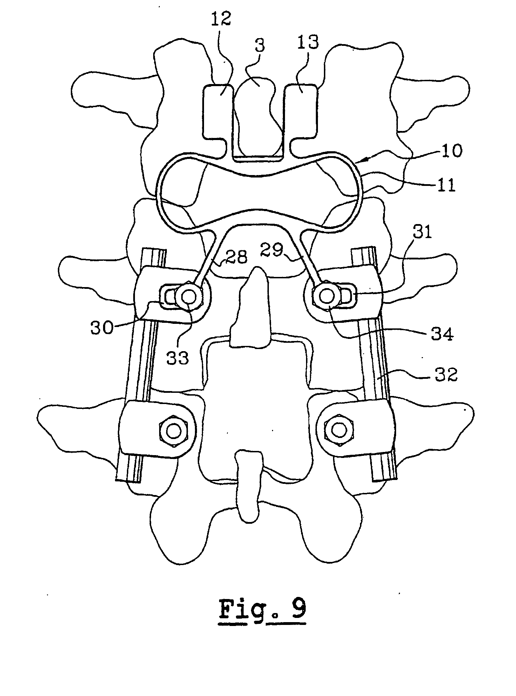

On a représenté en relation avec les figures 8 et 9, une autre forme de réalisation de l'invention. Pour l'essentiel, le corps (11) est sensiblement identique à celui illustré en relation avec les figures précédentes, mais ne comporte que deux pattes (12), (13) d'ancrage sur l'apophyse épineuse d'une vertèbre lombaire.

En revanche, les pattes (16) et (17) sont remplacées par deux pattes (28, 29), sensiblement en forme d'arc divergent, et dont les deux extrémités libres sont pourvues d'un orifice traversant (30, 31), destiné à permettre l'insertion à ce niveau de vis de fixation. Ces orifices traversants sont orientés non plus perpendiculairement au plan général des apophyses épineuses, mais parallèlement à ce plan.

Cette forme de réalisation est par exemple destinée à la zone particulière de la jonction entre les vertèbres lombaires et le sacrum, et notamment entre la lombaire L5 et la première vertèbre du sacrum S1. En effet, à ce niveau, la vertèbre S1 est dépourvue d'apophyse épineuse, de sorte qu'afin de permettre une stabilisation du rachis à ce niveau, il est nécessaire de prévoir un autre mode de fixation, tel que par le biais de l'arc divergent précité.

Cet arc divergent peut en outre présenter un segment beaucoup plus important, pour permettre l'implantation de l'implant vertébral inter-épineux entre une zone du rachis présentant un système de fixation de type arthrodèse, donc rigide, et une zone sous-jacente. On a ainsi représenté sur la figure 9, la mise en place de cet implant entre une vertèbre lombaire et une arthrodèse (32). Selon cette configuration, les orifices (30, 31) situés respectivement à l'extrémité des pattes (28) et (29), viennent s'enficher sur les vis pédiculaires respectives (33, 34) de la zone supérieure de l'arthrodèse, et sont maintenues à ce niveau au moyen d'un écrou ou système équivalent.

En d'autres termes, les orifices (30, 31) dont sont pourvues les deux extrémités libres de l'arc, sont destinées à recevoir les vis pédiculaires (33, 34) de l'arthrodèse considérée, pourvue à cet effet d'une extrémité libre, munie d'un filetage adapté.

Ce faisant, l'implant permet de lutter contre le syndrome jonctionnel bien connu lorsque l'on met en oeuvre des systèmes arthrodèse.

On conçoit dès lors tout l'intérêt de la présente invention, dans la mesure où l'implant vertébral permet d'assurer une stabilisation locale du rachis au niveau de son lieu d'implantation, tout en mettant en oeuvre une chirurgie peu invasive très rapide, évitant en outre tout risque d'éjection de l'implant lors des différents mouvements du rachis, notamment lors des mouvements de flexion.

## Revendications

1. Implant vertébral inter-épineux comportant :
- un corps (11) compressible selon la direction du rachis, constitué d'une boucle simple refermée sur elle-même, et destiné à venir s'insérer entre deux apophyses inter-épineuses consécutives (3, 4),
- ledit corps (11) étant muni de moyens d'ancrage au niveau d'au moins une apophyse épineuse constitués de deux pattes de fixation (12, 13), solidarisées au corps (11), s'étendant de part et d'autre de l'apophyse épineuse au niveau de laquelle ils sont destinés à venir s'ancrer lorsque l'implant est en place, et percées chacune d'un orifice traversant (14, 15), orienté selon une direction sensiblement perpendiculaire au plan général de ladite apophyse,
**caractérisé en ce que** :
- ledit implant comprend des plots de fixation (23), qui comportent donc tout d'abord une zone d'insertion (24), en forme de cône morse et de forme complémentaire à la forme interne des orifices traversants (14, 15), cette zone en forme de cône morse (24) se prolongeant par une zone cylindrique (25), de diamètre inférieur, se terminant par un profilé en forme de pointe (26), afin de permettre le sertissage dudit plot dans l'apophyse épineuse, et corollairement la fixation de l'implant,
- lesdits orifices traversants (14,15) étant destinés à recevoir lesdits plots de fixation (23) pour être sertis au niveau de l'apophyse considérée.

2. Implant vertébral inter-épineux selon la revendication 1, ***caractérisé* en ce que** le corps (11) est constitué d'une lame ressort fermée sur elle-même, sensiblement en forme de 8 ou de haricot, et symétrique par rapport au plan médian.

3. Implant vertébral inter-épineux selon l'une des revendications 1 et 2, ***caractérisé* en ce que** les moyens d'ancrage sont constitués par des pattes de fixation (12, 13, 16, 17), également symétriques l'une de l'autre par rapport au plan médian du corps (11), voire même parallèle l'une à l'autre, lesdites pattes de fixation présentant au voisinage de leur extrémité libre, un orifice traversant (14, 15, 18, 19), de forme tronconique, faisant fonction de cône morse, propre à coopérer avec des plots d'ancrage (23) de forme complémentaire, assurant ainsi leur autorétention à ce niveau, une fois les plots sertis dans l'apophyse épineuse.

4. Implant vertébral inter-épineux selon l'une des revendications 1 à 3, ***caractérisé* en ce que** le corps (11) comprend deux jeux de deux pattes de fixation pour permettre ainsi la fixation de l'implant vertébral inter-épineux au niveau des apophyses correspondantes de deux vertèbres consécutives.

5. Implant vertébral inter-épineux selon l'une des revendications 1 à 3, ***caractérisé* en ce que** le corps (11) comprend un premier jeu de deux pattes de fixation (12, 13) sur une apophyse épineuse, et un second jeu de pattes de fixation (28, 29), sensiblement en forme d'arc divergent, et dont les deux extrémités libres sont chacune pourvues d'un orifice traversant (30, 31), destiné à permettre l'insertion à ce niveau de vis de fixation (33, 34).

6. Implant vertébral inter-épineux selon la revendication 5, ***caractérisé* en ce que** les orifices traversants (30, 31) sont orientés parallèlement au plan général des apophyses épineuses.

## Patentansprüche

1. Interspinales Wirbelsäulenimplantat mit:
- einem in Richtung der Wirbelsäule kompressiblen Körper (11), der durch eine einfache, geschlossene Schlaufe gebildet wird und dazu bestimmt ist, interspinal zwischen zwei aufeinanderfolgenden Dornfortsätzen (3,4) eingefügt zu werden,
- wobei der genannte Körper (11) mit Mitteln zur Verankerung im Bereich mindestens eines Dornfortsatzes versehen ist, die von zwei fest mit dem Körper (11) verbundenen Befestigungslaschen (12,13) gebildet werden, welche sich zu beiden Seiten des Dornfortsatzes erstrecken, an dem sie zur Verankerung vorgesehen sind, wenn das Implantat an Ort und Stelle ist, und welche jeweils von einem Durchgangsloch (14,15) durchbrochen sind, das in einer Richtung im wesentlichen rechtwinklig zur Hauptebene des genannten Dornfortsatzes ausgerichtet ist,
**dadurch gekennzeichnet, daß**:
- das genannte Implantat Befestigungsstifte (23) umfaßt, die zunächst eine Einsatzzone (24) in Form eines Morsekonus und mit zur inneren Form der Durchgangslöcher (14,15) komplementärer Form aufweisen, wobei diese Zone in Form eines Morsekonus (24) von einer zylindrischen Zone (25) mit kleinerem Durchmesser verlängert wird, die in einem Profil in Form einer Spitze (26) endet, um eine Einpreßverbindung des genannten Stiftes in dem Dornfortsatz und folglich die Befestigung des Implantats zu ermöglichen,
- wobei die genannten Durchgangslöcher (14,15) dazu bestimmt sind, die genannten Befestigungsstifte (23) aufzunehmen, um im Bereich des betroffenen Dornfortsatzes eingepreßt zu werden.

2. Interspinales Wirbelsäulenimplantat nach Anspruch 1, **dadurch gekennzeichnet, daß** der Körper (11) von einem geschlossenen Federband im wesentlichen in Form einer 8 oder einer Bohne und symmetrisch in bezug auf die Mittelebene gebildet ist.

3. Interspinales Wirbelsäulenimplantat nach einem der Ansprüche 1 und 2, **dadurch gekennzeichnet, daß** die Verankerungsmittel durch Befestigungslaschen (12,13, 16,17) gebildet sind, die ebenfalls zueinander symmetrisch in bezug auf die Mittelebene des Körpers (11) und sogar zueinander parallel sind, wobei die genannten Befestigungslaschen nahe ihres freien Endes ein Durchgangsloch (14,15,18,29) mit kegelstumpfartiger Form aufweisen, das die Funktion eines Morsekonus erfüllt und dazu geeignet ist, mit Verankerungsstiften (23) mit komplementärer Form zusammenzuwirken, welche so ihre Selbsthemmung in diesem Bereich sicherstellen, wenn die Stifte in den Dornfortsatz eingepreßt sind.

4. Interspinales Wirbelsäulenimplantat nach einem der Ansprüche 1 bis 3, **dadurch gekennzeichnet, daß** der Körper (11) zwei Befestigungslaschen-Sätze aufweist, um so die Befestigung des interspinalen Wirbelsäulenimplantats im Bereich der entsprechenden Dornfortsätze der zweier aufeinanderfolgender Wirbel zu erlauben.

5. Interspinales Wirbelsäulenimplantat nach einem der Ansprüche 1 bis 3, **dadurch gekennzeichnet, daß** der Körper (11) einen ersten Satz mit zwei Laschen (12,13) zur Befestigung an einem Dornfortsatz und einen zweiten Satz mit Befestigungslaschen (28,29) im wesentlichen in Form eines divergierenden Bogens aufweist, deren zwei freie Enden jeweils mit einem Durchgangsloch (30,31) versehen sind, welches dazu bestimmt ist, das Einsetzen von Befestigungsschrauben (33,34) in diesem Bereich zu ermöglichen.

6. Interspinales Wirbelsäulenimplantat nach Anspruch 5, **dadurch gekennzeichnet, daß** die Durchgangslöcher (30,31) parallel zur Hauptebene der Dornfortsätze ausgerichtet sind.

## Claims

1. Interspinous vertebral implant comprising a body (11) compressible in the direction of the spine, formed by a single loop closed upon itself, and intended to be inserted between two consecutive interspinous processes (3, 4), said body (11) being provided with means for anchoring it to at least one spinous process consisting of two fixing lugs (12, 13), which are integral with the body (11), and extending on either side of the spinous process in the area of which they are intended to anchor when the implant is in place, and being each traversed by a through-opening (14, 15) oriented in a direction substantially perpendicular to the general plane of said process,
**characterized in that** :
• said implant comprises fixing studs (23), which thus first comprise an insertion zone (24) in the form of a morse cone and with a shape complementing the internal shape of the through-openings (14, 15), this zone in the form of a morse cone (24) being continued by a cylindrical zone (25), of smaller diameter, which ends in a pointed profile (26) in order to permit crimping of said stud in the spinous process and, by this means, fixation of the implant ;
• said through-openings being intended to receive said fixing studs (23) to be crimped in the area of the process in question.

2. Interspinous vertebral implant according to claim 1, wherein the body (11) is formed by a spring blade closed upon itself, substantially in the shape of a figure of 8 or kidney bean, and symmetrical with respect to the median plane.

3. Interspinous vertebral implant according to any of claims 1 and 2, wherein the anchoring means are formed by fixing lugs (12, 13, 16, 17), also mutually symmetrical with respect to the median plane of the body (11), even parallel to one another, said fixing lugs having, near their free end, a through-opening (14, 15, 18, 19) of truncated cone shape functioning as a morse cone able to cooperate with anchoring studs (23) of complementary shape, thus ensuring their retention in this area once the studs have been crimped in the spinous process.

4. Interspinous vertebral implant according to anyone of claims 1 to 3, wherein the body (11) comprises two sets of two fixing lugs in order to thus permit fixation of the interspinous vertebral implant in the area of the corresponding processes of two consecutive vertebrae.

5. Interspinous vertebral implant according to anyone of claims 1 to 3, wherein the body (11) comprises a first set of two fixing lugs (12, 13) on a spinous process, and a second set of fixing lugs (28, 29) which are substantially in the form of a divergent arc and whose two free ends are each provided with a through-opening (30, 31) intended to permit insertion of fixing screws (33, 34) in this area.

6. Interspinous vertebral implant according to claim 5, wherein the through-openings (30, 31) are oriented parallel to the general plane of the spinous processes.
